# EUROPÄISCHE PATENTANMELDUNG

(11) **EP 3 578 225 A1**
(43) Veröffentlichungstag der Anmeldung: **11.12.2019**
(21) Anmeldenummer: 19175310.2
(22) Anmeldetag: 20.05.2019
(51) Int. Cl.: A61M 37/00

(54) **ANTRIEB FUER MIKRONADELVORRICHTUNG UND MIKRONADELVORRICHTUNG**

(30) Priorität: 04.06.2018 DE 102018113210
(71) Anmelder: Promedia Gesundheitspartner GmbH, 69115 Heidelberg (DE)
(72) Erfinder: Arnold, Werner, 69115 Heidelberg (DE)
(74) Vertreter: Weber & Seidel

(57) **Zusammenfassung**

Antrieb (2) einer Mikronadelvorrichtung (1) mit einem Rotationsantrieb (3) und einer Wandlungseinrichtung (4) zur Wandlung der Rotations- (5) in Hubbewegungen (6), welche über eine Pleuelstange (8) und eine Antriebsstange (9) vermittelt werden, wobei der Antrieb (2) und die Wandlungseinrichtung (4) in einem stabförmigen Modulgehäuse (10) angeordnet sind.

Stift (12) beschreibt bei der Rotation eine Kreisbewegung (13) und greift in eine Wandlungswippe (14) ein, die mit einer senkrecht zur Rotationsachse (11) angeordneten Schwenkachse (15) gelagert ist, wobei der Stift (12) derart eingreift, daß er in der Richtung des Verlaufs der Schwenkachse (15) in der Wandlungswippe (14) gleitet, aber in Schwenkrichtung dieser eine Schwenkbewegung (16) vermittelt und daß die Pleuelstange (8) ebenfalls beabstandet von der Schwenkachse (15) an die Wandlungswippe (14) angelenkt ist, wobei eine weitere Anlenkung an die Antriebsstange (9), die längs geführt ist, der Übertragung der Schwenkbewegung (16) und Umwandlung in die Hubbewegung (6) dient.

## Beschreibung

Die Erfindung betrifft einen Antrieb für Mikronadelvorrichtung mit einem Rotationsantrieb und einer Wandlungseinrichtung zur Wandlung der Rotationsbewegung in Hubbewegungen für Mikronadeln, welche diesen über eine Pleuelstange und eine daran angelenkte Antriebsstange vermittelt werden, wobei der Antrieb und die Wandlungseinrichtung in einem stabförmigen Modulgehäuse angeordnet sind.

Die Erfindung betrifft weiterhin eine Mikronadelvorrichtung mit einem solchen Antrieb.

Mikronadelvorrichtungen dienen sowohl dem Tätowieren als auch der physikalischen Hautbehandlung. Bei letzterem werden feine, das heißt atraumatische, Nadeln von 0,1 bis ca. 0,25 mm Durchmesser in die obere Schicht der Haut, die Epidermis, eingestochen, um winzige Verletzungen zu erzeugen, die so gering sind, da sie keine Narben bilden. Dies stimuliert die Hautregeneration, was eine Hautverbesserungen bei Narben, Pigmentstörungen, Falten und ähnlichen Hautproblemen bewirkt. Durch die erzeugten Mikroverletzungen werden u.a. hauteigene Wachstumsfaktoren freigesetzt, Kollagenfasern und Blutgefäße neu gebildet und die Zuwanderung von Fibroblasten initiiert. Dabei bestimmt die Einstechtiefe darüber, ob es sich um eine kosmetische Behandlung (bis 0,5 mm) handelt oder um eine medizinische (über 0,5 mm).

Da die Haut an den verschiedenen Körperpartien eine unterschiedliche Dicke aufweist, ist die Einstichtiefe, das heißt der Überstand der Nadel vom Gehäuse bei der Vorschubbewegung, unabhängig von der Frage, ob die Vorrichtung zu medizinischen oder zu kosmetischen Behandlungen eingesetzt wird, von großer Bedeutung. Beispielsweise ist die Haut im Gesicht und auf dem Handrücken dünner als an anderen Körperpartien. Aber auch Narbengewebe kann eine dickere Hautstruktur bilden, so daß es wichtig ist, die Nadeleinstichtiefe variabel einstellen zu können, um sie an die unterschiedlich dicken Epidermisschichten anpassen zu können, die zwischen 0,03 mm und 2 mm variieren, im Gesichtsbereich mit besonders dünner Haut sogar nur zwischen 0,03 mm und 0,2 mm. Die Nadeleinstichtiefe bestimmt sich über den Überstand der Nadeln über das Gehäuse bei ausgefahrenem Hub.

Aus der EP 2 954 925 A1 ist eine Mikronadelvorrichtung bekannt, bei der ein rotierender Stift seitlich in ein Langloch eines Pleuelgestänges eingreift und so die Rotation des seitlich des Pleuelgestänges angeordneten Motors in die Hubbewegung wandet. Auf diese Weise läßt sich allerdings keine stabförmig ausgebildete Mikronadelvorrichtung realisieren, da der Motor seitlich auskragt. Viele Anwender bevorzugen jedoch ein stabförmig ausgebildetes Gerät, weil sie es gezielter über den Einsatzbereich führen können.

Bei der oben genannten Mikronadelvorrichtung erfolgt die Hubverstellung durch Verstellung des Anlenkungspunktes der angetriebenen Pleuelstange am Gehäuse. Dadurch wird entsprechend der Veränderung des Hubs durch die Veränderungen der Hebellängen die Bewegungsgeschwindigkeit der Nadeln verstellt, da die Drehzahl des Motors gleich bleibt. So führt beispielsweise eine Verdopplung der Hublänge zu einer Halbierung der Nadelgeschwindigkeit. Der Hub findet bei dieser Vorrichtung unabhängig von der Hublänge außerhalb des Gehäuses statt, wobei ein großer Hub schnell und ein kleiner Hub entsprechend langsamer vollzogen wird. Da das Gerät auf der Haut dabei verschoben wird, kommt es außer dem Einstechen in die Haut beim Verfahren der Mikronadelvorrichtung zu einem Ritzen der Haut, was eine größere Verletzung darstellt. Dies ist insbesondere bei kleinen Hüben für dünne Hautbereiche besonders problematisch, da sich die Nadeln dabei auch noch mit geringerer Geschwindigkeit bewegen und es keine nennenswerte Zeitspanne gibt, während der sie ganz in das Gehäuse zurückgezogen sind. Durch das Verschieben eingestochener Nadeln wird die Haut geritzt. Will man dies verhindern, muß man die Vorrichtung beabstandet von der Haut führen, dann ist aber auch die Einstichtiefe nicht mehr kontrollierbar. Insbesondere bei kosmetischer Behandlung und Gesichtshaut wird es aber wenig akzeptiert, wenn die Haut durch das Ritzen nach einer Behandlung längere Zeit gerötet ist. Bei medizinischer Behandlung, insbesondere bei dicken Hautstrukturen, wie Narbenbehandlung, wird dies dagegen eher akzeptiert, zumal dies meist Bereiche sind, auf denen Kleidung getragen wird.

Diese Probleme sind weitgehend entschärft, wenn man nach Vorschlägen des Standes der Technik die Mikronadelvorrichtung mit einem Antrieb versieht, der immer den gleichen Hub mit immer der gleichen Nadelgeschwindigkeit ausführt, dafür aber der ausgeführte Hub gegenüber der Nadelaustrittsöffnung - oder diese gegenüber dem ausgeführten Hub - derart verschoben wird, daß der wirksame Hub durch den eingestellten Überstand der Nadeln über die Nadelaustrittsöffnung bestimmt wird. Statt der Veränderung der Geschwindigkeit und der von der Mechanik erzeugten Hublänge wird hier der Nadelhub je nach eingestellter wirksamer Hubgröße mehr oder weniger in das Gehäuse verlagert. Die Geschwindigkeit der Nadeln bleibt dabei gleich, aber der wirksame Nadelaustritt wird verändert, was dazu führt, daß bei kleinem Hub für dünne Haut kurz eingestochen wird und dann eine Pause folgt, in der die Nadeln in das Gehäuse zurückgezogen sind. In dieser Zeit kann die Mikronadelvorrichtung ohne unnötiges Aufreißen der Haut weitergeführt werden. Damit wird bei medizinischer Behandlung und dicker Haut in Kauf genommen, daß die Haut durch die längere Verweilzeit der Nadeln in der Haut und geringerer Zeit, in der sie in das Gehäuse zurückgezogen sind, stärker strapaziert wird. Bei medizinischer Behandlung und dicker Haut wird dies jedoch - wie oben schon ausgeführt - akzeptiert. Anders ist dies bei dünner Haut und bei kosmetischen Behandlungen. Bei diesen Behandlungen ist die Verweilzeit der Nadeln in der Haut bei der vorgenannten Vorrichtung sehr kurz. Zwischen zwei Hüben entsteht eine Verweilzeit der Nadeln im Gerät, während der dieses, ohne die Haut zu belasten, verschoben werden kann. Die kurze Verweilzeit der Nadeln in der Haut bei den kleinen Hüben führt in der Regel dazu, daß die Elastizität der Haut ausreicht, jegliches Einreißen zu vermeiden. Die Vorrichtung kann dann auf der Haut aufliegen, und der wirksame Hub ist dadurch exakt unter Kontrolle.

Beispiele für solche Mikronadelvorrichtungen sind die CN 202236855 U1 und die DE 10 2016 106 042 A1. Die Erfindung will die beschriebene vorteilhafte Funktion dieser Geräte beibehalten, aber die Wandlungseinrichtung verbessern.

Bei der CN 202236855 U1 wurde diese vorteilhafte Verstellung des wirksamen Hubs mit einem Antrieb der eingangs genannten Art gelöst, wobei durch die Ausgestaltung ein Antrieb realisiert wurde, bei dem der Motor nicht seitlich auskragt, sondern in Längsrichtung des Geräts derart angeordnet ist, daß die Mikronadelvorrichtung als stabförmiges Gerät ausgeführt werden kann. Allerdings weist die Wandlungseinrichtung zur Wandlung der Bewegung von einer Rotationsbewegung zu einem Hub bei diesem Antrieb einen Schlitten auf, der oben eine pfannenförmige Vertiefung hat, in die ein sich exzentrisch bewegender Bolzen eingreift, um die Drehung des Motors in eine Hin - und Herbewegung zu wandeln. Dieser Schlitten ist wegen der erforderlichen hohen Geschwindigkeit zur Erzielung einer schnellen Hubbewegung einem hohen Verschleiß unterworfen, da sowohl der Bolzen in der pfannenförmigen Vertiefung reibt, als auch der Schlitten auf einer Bahn gleitet, welche die Gegenkraft aufbringen muß, um den exzentrisch rotierenden Bolzen in der Pfanne zu halten.

Das oben genannte Problem wird auch von der DE 10 2016 106 042 A1 mit einem Antrieb der eingangs genannten Art gelöst. Bei diesem Antrieb wird die Umwandlung der rotierenden Bewegung des Motors in eine Hubbewegung durch ein Schwenkelement gelöst, in das ein Exzenter eingreift. Das Schwenkelement weist dabei zwei Anschläge auf, die durch den Exzenter abwechselnd mit Kräften für die Schwenkung beaufschlagt werden . Dadurch sind die zwei Anschläge durch die Reibkräfte des mit hoher Geschwindigkeit rotierenden Exzenters einer starken Wechselbeanspruchung ausgesetzt. An den Anschlägen reibt der Exzenter nicht nur, sondern die Anschläge führen dabei auch noch eine quer zur reibenden Rotationsbeanspruchung verlaufende Kippbewegung aus, was ein zusätzliches reibendes Hin- und Hergleiten zwischen Anschlägen und Exzenter unter hochfrequenter Wechselbeanspruchung darstellt. Das Schwenkelement ist daher hohem Verschließ ausgesetzt.

Der Erfindung liegt daher die Aufgabe zugrunde, einen Antrieb der eingangs genannten Art derart auszugestalten, daß der Verschleiß möglichst gering gehalten wird.

Die Aufgabe wird dadurch gelöst, daß am Ende der Achse des Rotationsantriebs ein Stift derart angeordnet ist, daß er bei der Rotation eine Kreisbewegung beschreibt und in eine Wandlungswippe eingreift, die mit einer senkrecht zur Rotationsachse angeordneten Schwenkachse am Modulgehäuse gelagert ist, wobei der Stift in die Wandlungswippe beabstandet von der Schwenkachse derart eingreift, daß er in der Richtung des Verlaufs der Schwenkachse in der Wandlungswippe gleitet, aber in Schwenkrichtung dieser eine Schwenkbewegung vermittelt, und daß die Pleuelstange ebenfalls beabstandet von der Schwenkachse an die Wandlungswippe angelenkt ist, wobei eine weitere Anlenkung an die Antriebsstange, die längs geführt ist, der Übertragung der Schwenkbewegung und Umwandlung in die Hubbewegung dient.

Der Vorteil dieser Ausgestaltung besteht zum einen darin, daß der Rotationsantrieb, dessen Achse in Richtung der Hubbewegung ausgerichtet ist, eine Anordnung des Rotationsantriebs in Verlängerung der Hubbewegung erlaubt und damit auch eine stabförmige Mikronadelvorrichtung möglich wird. Im Unterschied zur DE 10 2016 106 042 A1 wurde der Exzenter durch einen eine Kreisbewegung beschreibenden Stift ersetzt, der durch seinen geringen Durchmesser bei gleicher Huberzeugung eine wesentlich geringere Reibfläche aufweist, so daß sowohl an dem Stift als auch an der Wandlungswippe die Reibfläche verringert ist. Der Eingriff des Stifts in die Wandlungswippe ist einerseits eine hin- und hergleitende Bewegung und andererseits eine wechselweise Druckausübung, die die hin- und hergehende Wippbewegung der Wandlungswippe erzeugt. Das Gleiten des Stifts kann allerdings noch dadurch vermieden werden, daß der Stift drehbar gelagert ist und dadurch eine Rollbewegung in einer Führung für den Stift in der Wippe entsteht.

Diese Führung kann so ausgestaltet sein, daß der Eingriff des Stifts in die Wandlungswippe ein in derselben Richtung wie die Schwenkachse ausgerichtetes Langloch ist, in dem der Stift gleitet oder rollt. Eine andere Möglichkeit besteht darin, daß der Stift in eine Bohrung eines in der Wandlungswippe gelagerten Schiebestifts eingreift, wobei der Schiebestift, der in derselben Richtung wie die Schwenkachse verläuft, verschiebbar in der Wandlungswippe gelagert ist. Der Schiebestift kann als runder Stift oder als ein hin- und herbewegbares Kulissenelement ausgebildet sein. In allen Fällen ist die Reibung geringer als bei den beiden vorgenannten Konstruktionen des Standes der Technik.

Vorzugsweise ist der Antrieb derart ausgestaltet, daß die Schwenkachse der Wandlungswippe, der Eingriff des Stifts und die Anlenkung der Pleuelstange ein Dreieck bilden. Die beiden von der Schwenkachse ausgehenden Dreiecksseiten bilden Hebel, wobei der zwischen Schwenkachse und Eingriff des Stifts verlaufende Hebel der Erzeugung der Schwenkbewegung der Wandlungswippe dient und der zwischen Schwenkachse und Anlenkung der Pleuelstange verlaufende Hebel der Übertragung der Schwenkbewegung auf die Pleuelstange. Die an die Pleuelstange angelenkte Antriebsstange ist dann mit einer geraden Führung versehen und vollzieht die reine Hin- und Herbewegung des Hubs, der den Nadeln weitervermittelt wird.

Die Erfindung betrifft weiterhin eine Mikronadelvorrichtung mit einem Antrieb der vorgenannten Art. Bei dieser ist das stabförmige Modulgehäuse in einem stabförmigen Vorrichtungsgehäuse angeordnet, so daß eine Mikronadelvorrichtung in Stabform gebildet ist. Die Einstellung des Hubs erfolgt dadurch, daß eine Stichtiefeneinstellhülse durch ein Gewinde derart mit dem Vorrichtungsgehäuse in Wirkverbindung steht, daß durch Drehung der Stichtiefeinstellhülse der Austrittshub der Mikronadeln aus der Nadelaustrittsöffnung einstellbar ist.

Es wird somit sowohl die von vielen Anwendern gewünschte Stabform realisiert, als auch eine Hubeinstellung, bei der der Antrieb immer die gleichen Hubbewegungen erzeugt, der wirksame Hub aber dadurch eingestellt wird, daß sich der Austrittshub der Nadeln aus der Nadelaustrittsöffnung mittels der Stichtiefeneinstellhülse verändern läßt. Der Austrittshub entspricht dann der Stichtiefe der Nadeln in die Haut. Da die Nadelgeschwindigkeit unabhängig von der Stichtiefe immer gleich ist und auch die Zeit von einem Einstich zum nächsten - unabhängig von der Hubgröße - immer gleich ist, kann der Anwender leicht ein Gefühl dafür entwickeln, mit welcher Geschwindigkeit er das Gerät auf der Haut fortbewegen muß, um gleichmäßig verteilte Nadeleinstiche auf der Haut zu erzielen. Bei geringer Einstichtiefe wird - durch die kurze Verweilzeit der Nadeln in der Haut mit den längeren Zeiten des Rückzugs der Nadeln in das Gehäuse - die Haut durch ihre Elastizität befähigt, das Verschieben der Vorrichtung während der Einstichzeit insoweit auszugleichen, daß es zu keinem Einreißen kommt. Wird dagegen dickere Haut mit entsprechend großer Einstichtiefe bearbeitet, so kann diese weit mehr Belastungen ausgleichen - sie reißt daher meist auch bei längerer Nadelverweilzeit in der Haut und einem Verschieben der Vorrichtung nicht so schnell ein. Wenn dies bei maximaler Einstichtiefe und intensiver medizinischer Behandlung dicker Haut, wie beispielsweise Narben, doch passiert, ist es akzeptabler, da solche Hautpartien normalerweise von Kleidung bedeckt sind oder bei medizinischer Behandlung auch ein kurzzeitiger Verband akzeptiert wird.

Der wirksame Hub läßt sich sowohl dadurch einstellen, daß der gesamte Antrieb mit den Nadeln innerhalb des Modulgehäuses verschoben wird oder dadurch, daß der vordere Teil des Vorrichtungsgehäuses mit der Nadelaustrittsöffnung gegenüber dem hinteren Teil verschoben, sozusagen das Gehäuse verkürzt oder verlängert wird, womit auch der Nadelaustritt aus der Nadelaustrittsöffnung verkürzt oder verlängert wird. Beispielsweise kann vorgesehen sein, daß die Mikronadelvorrichtung derart ausgestaltet ist, daß der Antrieb mit einem hinteren Teil des Vorrichtungsgehäuses verbunden ist und daß die Stichtiefeeinstellhülse auf einen vorderen Teil des Vorrichtungsgehäuses wirkt, das mit dem hinteren Teil des Vorrichtungsgehäuses verschiebbar aber drehfest verbunden ist. Wenn dann das vordere Teil des Vorrichtungsgehäuses so ausgebildet ist, daß es ein Nadelführungsteil und einen darin verschiebbar, aber drehfest gelagerten Nadelhalter aufweist, so ist es auch möglich, daß der Nadelhalter und die Nadelaustrittsöffnung für in einer Reihe angeordneten Mikronadeln ausgebildet sind. Solche Reihen von Nadeln dienen der Behandlung von größeren Hautflächen, wobei die Vorrichtung quer zur Nadelreihe über die Haut gezogen wird und so Streifen in der Breite der Nadelreihe behandelbar sind, die so lange nebeneinander gelegt werden, bis die zu behandelnde Hautpartie abgearbeitet ist.

Die Mikronadelvorrichtung ist vorzugsweise derart ausgebildet, daß das Nadelführungsteil und der Nadelhalter mit den Mikronadeln in einem Auswechselmodul angeordnet und an der übrigen Mikronadelvorrichtung lösbar befestigt sind, wobei der Nadelhalter mit der Antriebsstange mittels einer Kupplung verbindbar ist.

Die vorgenannten Ausgestaltungen mit in einer Reihe angeordneten Nadeln und deren Führung im Vorrichtungsgehäuse, als auch die Ausgestaltung des Auswechselmoduls mit der Kupplung der Antriebsstange mit dem Nadelhalter sind aus der DE 20 2018 100 639 U1 bekannt. Wenn dort diese Elemente auch nicht anhand eines Geräts mit stabförmigem Modulgehäuse sowie mit einem anderen Antrieb beschrieben sind, so sind die vorerwähnten Elemente doch ohne weiteres auf die erfindungsgemäße Mikronadelvorrichtung übertragbar. Insoweit wird eine nähere Beschreibung durch diesen Verweis ersetzt.

Die Erfindung wird nachfolgend anhand von in der Zeichnung dargestellten Ausführungsbeispielen erläutert. Es zeigen
- **Fig. 1**: ein Ausführungsbeispiel eines Antriebs mit Wandlungsvorrichtung für eine Mikronadelvorrichtung,
- **Fig. 2a, 2b und 2c**: ein weiteres Ausführungsbeispiel eines Antriebs mit Wandlungsvorrichtung für eine Mikronadelvorrichtung in verschiedenen Funktionsstellungen,
- **Fig. 3**: eine Mikronadelvorrichtung mit Stichtiefeeinstellhülse,
- **Fig. 4**: einen Längsschnitt durch eine Mikronadelvorrichtung mit Stichtiefeeinstellhülse und
- **Fig. 5**: einen Längsschnitt durch ein Auswechselmodul mit einer Reihe von Mikronadeln.

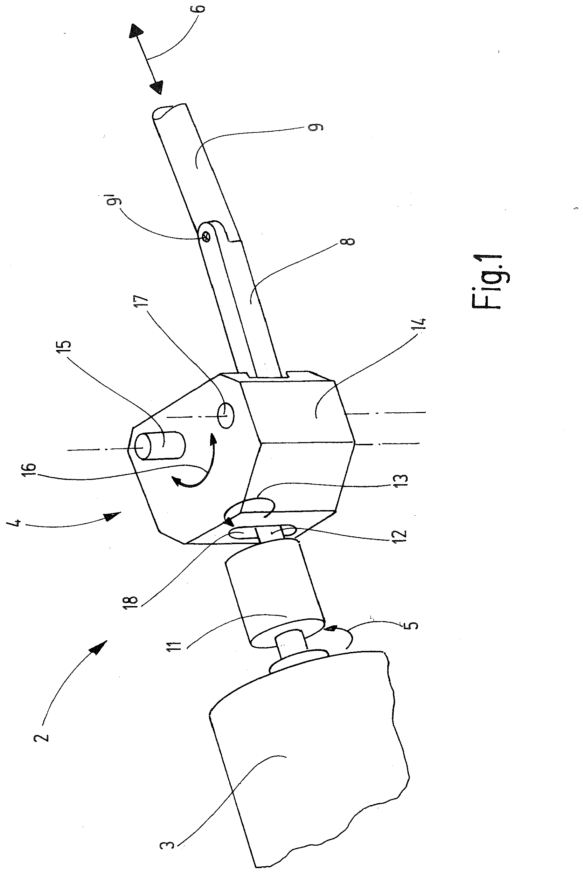

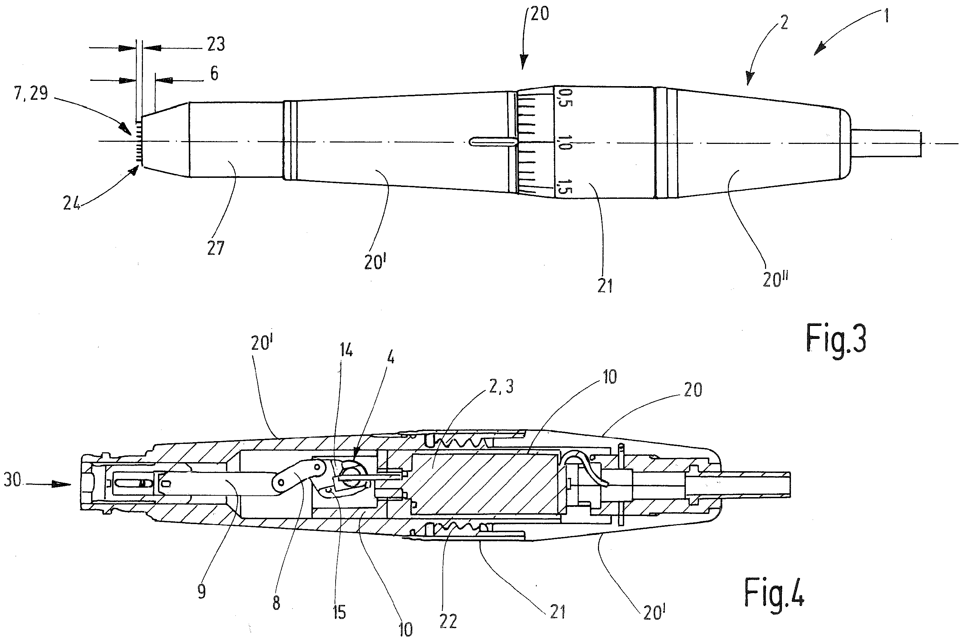

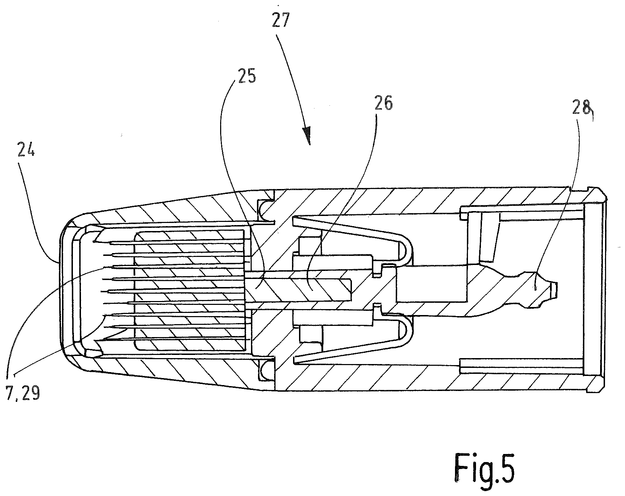

**Fig. 1** zeigt ein Ausführungsbeispiel eines Antriebs 2 mit einer Wandlungsvorrichtung 4 für eine Mikronadelvorrichtung 1. Die Wandlungsvorrichtung 4 dient dazu, eine Rotationsbewegung 5 eines Rotationsantriebs 3, beispielsweise eines Elektromotors, in eine Hubbewegung 6 für Mikronadeln 7 zu wandeln. Zu diesem Zweck ist auf der Achse 11 des Rotationsantriebs 3 ein Stift 12 derart angeordnet, daß er eine Kreisbewegung 13 beschreibt. Der Stift 12 greift in ein Langloch 18 einer Wandlungswippe 14 ein, bewegt sich durch die Kreisbewegung 13 in dem Langloch 18 auf und ab und vermittelt der Wandlungswippe 14 gleichzeitig eine Hin- und Herbewegung, so daß die Wandlungswippe 14 um ihre Schwenkachse 15 hin- und herschwenkt, wie dies der Doppelpfeil 16 zeigt. Die Schwenkachse 15 ist dabei in einem Modulgehäuse 10 gelagert (dies ist in Fig. 4 dargestellt). An die Wandlungswippe 14 ist eine Pleuelstange 8 angelenkt, wobei hier die Anlenkung 17 als Achse ausgebildet ist. Die Pleuelstange 8 bekommt von der Wandlungswippe 14 zwar schon die Hubbewegung 6 vermittelt, diese ist jedoch noch gleichzeitig mit einer schwenkenden Hin- und Herbewegung verbunden. Um eine reine Hubbewegung 6 erzielen, ist die Pleuelstange 8 mittels einer Anlenkung 9' mit einer Antriebsstange 9 verbunden, wobei die Antriebsstange 9 eine nicht gezeichnete Geradführung aufweist. Die Antriebstange 9 vermittelt ihre Hubbewegung 6 der oder den Mikronadeln 7 (siehe Fig. 3 und 5).

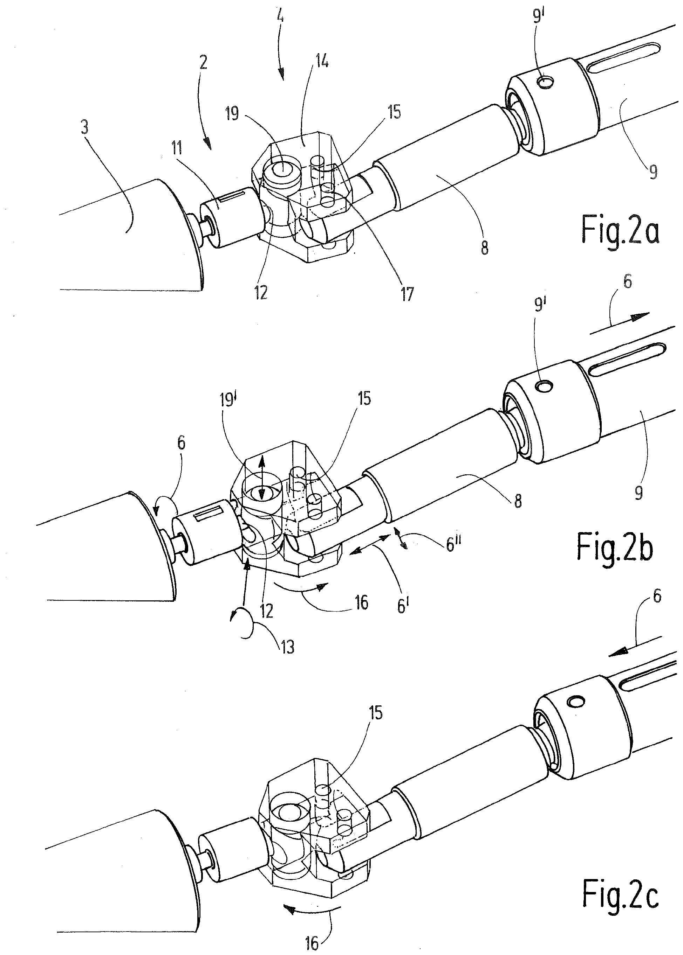

Die **Figuren 2a, 2b und 2c** zeigen ein weiteres Ausführungsbeispiel eines Antriebs 2 mit einer Wandlungsvorrichtung 4 für eine Mikronadelvorrichtung 1. Dabei zeigen die einzelnen Figuren unterschiedliche Funktionsstellungen der Wandlungsvorrichtung 4.

In diesen Darstellungen entsprechen dieselben Bezugszeichen wie in Fig. 1 denselben Funktionselementen mit denselben Funktionen. Der Unterschied zum Ausführungsbeispiel gemäß Fig. 1 besteht darin, daß der Stift 12 statt in ein Langloch 18 in einen Schiebestift 19 eingreift. Auf diese Weise wird, wie in Fig. 2b eingezeichnet, die Kreisbewegung 13 des Stifts 12 in eine Auf- und Abbewegung 19' des Schiebestifts 19 gewandelt - was funktional der Auf- und Abbewegung des Stifts 12 in dem Langloch 18 der Fig. 1 entspricht. Der Vorteil besteht darin, daß der Stift 12 bei diesem Ausführungsbeispiel in dem Schiebestift 19 nur noch eine reine Rotationsbewegung vollzieht und durch die Entkopplung von Rotationsbeanspruchung und dem Gleiten weniger Reibung als an den Langlochwandungen auftritt.

Auch bei diesem Ausführungsbeispiel schwenkt die Wandlungswippe 14 um die Schwenkachse 15, vermittelt dabei der angelenkten Pleuelstange 8 eine Bewegung, die sich - siehe Fig. 2b - aus einer Hubbewegung 6' und einer Schwenkbewegung 6" zusammensetzt, welche mittels der Anlenkung 9' der Antriebsstange 9 an die Pleuelstange 8 in eine reine Hubbewegung 6 umgewandelt wird, da die Antriebsstange 9 auch hier eine (nicht gezeichnete) Geradführung aufweist. Bei genauem Vergleich ist ersichtlich, daß der Schiebestift 19 in den Figuren 2a, 2b und 2c unterschiedliche Positionen in seiner Auf- und Abbewegung 19' aufweist, wodurch auch die Wandlungswippe 14 verschiedene mit den Pfeilen 16 angedeutete Schwenkpositionen einnimmt und dadurch die Hubbewegung 6 erzeugt wird.

**Fig. 3** zeigt eine Mikronadelvorrichtung 1 in Stabform mit einer Stichtiefeeinstellhülse 21, welche einen erfindungsgemäßen Antrieb 2 mit Wandlungsvorrichtung 4 enthält. In dem Vorrichtungsgehäuse 20 befindet sich das Modulgehäuse 10, in das Antrieb 2 und Wandlungsvorrichtung 4 eingebaut sind, wie dies in Fig. 4 dargestellt ist. Das Vorrichtungsgehäuse 20 besteht aus einem vorderen Teil 20', an das ein Auswechselmodul 27 angefügt ist, und einem hinteren Teil 20' des Vorrichtungsgehäuses 20, also nach der Stichtiefeneinstellhülse 21. In diesem hintere Teil 20" des Vorrichtungsgehäuses ist der als Rotationsantrieb 3 ausgebildete Antrieb 2 angeordnet.

Durch die Verstellung der Stichtiefeneinstellhülse 21 wird bestimmt, wieviel von der Hubbewegung 6 der Mikronadeln 7 als Austrittshub 23 vollzogen - und damit wirksam wird - und wieviel der Hubbewegung 6 sich innerhalb des Gehäuses der Mikronadelvorrichtung 1 vollzieht und dadurch nicht wirksam wird. Dadurch wird nicht nur die Stichtiefe bestimmt, sondern je nachdem, wie groß der Anteil des Austrittshubs 23 an dem Gesamthub 6 ist, wird auch bestimmt, wie groß die Zeiten sind, in denen die Nadeln 7 vollständig in das Vorrichtungsgehäuse 20 zurückgezogen sind, wie dies oben bereits beschrieben ist.

Die Mikronadeln 7, die hier in einer Reihe 29 angeordnet sind, befinden sich in einem Auswechselmodul 27, da aus hygienischen Gründen sowohl die Mikronadeln 7 als auch dieser vordere Bereich der Mikronadelvorrichtung 1 - also das Auswechselmodul 27 - bei jedem neuen Patienten auszuwechseln ist.

**Fig. 4** zeigt einen Längsschnitt durch eine Mikronadelvorrichtung 1 der vorgenannten Art mit einer Stichtiefeneinstellhülse 21. Die Stichtiefeneinstellhülse 21 weist dabei ein Gewinde 22 auf, das bei einer Drehung der Stichtiefeneinstellhülse 21 den vorderen Teil 20' des Vorrichtungsgehäuses 20 gegenüber dem hinteren Teil 20" derart verschiebt, daß der Austrittshub 23 der Mikronadeln 7 aus der Nadelaustrittsöffnung 24 größer oder kleiner wird.

In dieser Schnittdarstellung ist auch sichtbar, wie das Modulgehäuse 10 in das Vorrichtungsgehäuse 20 eingefügt ist und in diesem sich sowohl der Antrieb 2 als auch die Wandlungsvorrichtung 4 befinden, wobei die Wandlungswippe 14 mit der Schwenkachse 15 in dem Modulgehäuse 10 liegt. Da der vordere Teil 20' des Vorrichtungsgehäuses 20 relativ zu Antrieb 2 und Wandlungsvorrichtung 4 verschoben wird, kann auch der Austrittshub 23 eingestellt werden. Selbstverständlich können das Modulgehäuse 10 und der hintere Teil 20' des Vorrichtungsgehäuses 20 auch einteilig ausgeführt sein.

Bei der gezeichneten Schnittdarstellung befindet sich das Auswechselmodul 27 nicht auf der Mikronadelvorrichtung 1; der Pfeil 30 zeigt jedoch, wie das Auswechselmodul 27 auf die Mikronadelvorrichtung 1 aufgesteckt wird.

**Fig. 5** zeigt eine Schnittdarstellung eines solchen Auswechselmoduls 27 , das auf den vorderen Teil 20' des Vorrichtungsgehäuses 20 aufsteckbar ist und zu diesem Zweck eine Kupplung 28 für die Bewegungsübertragung der Hubbewegung 6 aufweist. In dem Auswechselmodul 27 ist ein Nadelhalter 25 verschiebbar gelagert, wobei ein Nadelführungsteil 26 derart ausgebildet ist, daß zwar eine Verschiebung aber keine Drehung stattfinden kann. Im vorderen Bereich sind die Mikronadeln 7, welche in einer Reihe 29 angeordnet sind, sichtbar. Sie befinden sich bei dieser Stellung gerade innerhalb des Vorrichtungsgehäuses 20, wobei sie im Sticheinsatz aus der Nadelaustrittsöffnung 24 entsprechend dem eingestellten Austrittshub 23 heraustreten.

Bezüglich der näheren Ausgestaltung dieses Auswechselmoduls 27 wird auf die DE 20 2018 100 639 U1 verwiesen, wobei das Auswechselmodul 27 und die Verbindung desselben mit der Mikronadelvorrichtung 1 auf die erfindungsgemäße Vorrichtung übertragbar ist. In der genannten Schrift ist allerdings der Antrieb mit der Wandlungsvorrichtung auf andere Weise ausgebildet, ebenso auch die Ausgestaltung der Stichtiefeneinstellung.

Eine Mikronadelvorrichtung 1 der erfindungsgemäßen Art kann selbstverständlich in verschiedenster Art und Weise ausgestaltet sein und es können auch unterschiedliche Methoden zur Einstellung des Austrittshubs 23 vorgesehen sein, wesentlich ist dabei lediglich, daß der Antrieb 2 auf eine Wandlungsvorrichtung 4 mit einer Wandlungswippe 14 derart wirkt, daß auch bei den äußerst hohen Geschwindigkeiten die Reibung derart vermindert ist, daß dies eine lange Einsatzdauer ermöglicht.

### Bezugszeichenliste

- 1: Mikronadelvorrichtung
- 2: Antrieb
- 3: Rotationsantrieb
- 4: Wandlungsvorrichtung
- 5: Pfeil: Rotationsbewegung
- 6: Pfeil: Hubbewegung
- 6': Hubbewegung der Pleuelstange
- 6": Schwenkbewegung der Pleuelstange
- 7: Mikronadeln
- 8: Pleuelstange
- 9: Antriebsstange
- 9': Anlenkung der Antriebsstange an die Pleuelstange
- 10: Modulgehäuse
- 11: Achse des Rotationsantriebs
- 12: Stift
- 13: Kreisbewegung
- 14: Wandlungswippe
- 15: Schwenkachse
- 16: Doppelpfeil: Schwenkbewegung
- 17: Anlenkung der Pleuelstange
- 18: Langloch
- 19: Schiebestift
- 19': Auf- und Abbewegung des Schiebestifts
- 20: Vorrichtungsgehäuse
- 20': vorderer Teil des Vorrichtungsgehäuses
- 20": hinterer Teil des Vorrichtungsgehäuses
- 21: Stichtiefeeinstellhülse
- 22: Gewinde
- 23: Austrittshub
- 24: Nadelaustrittsöffnung
- 25: Nadelhalter
- 26: Nadelführungsteil
- 27: Auswechselmodul
- 28: Kupplung
- 29: Reihe von Mikronadeln
- 30: Pfeil: Aufstecken und Einkuppeln des Auswechselmoduls

## Patentansprüche

1. Antrieb (2) für Mikronadelvorrichtung (1) mit einem Rotationsantrieb (3) und einer Wandlungseinrichtung (4) zur Wandlung der Rotationsbewegung (5) in Hubbewegungen (6) für Mikronadeln (7), welche diesen über eine Pleuelstange (8) und eine daran angelenkte Antriebsstange (9) vermittelt werden, wobei der Antrieb (2) und die Wandlungseinrichtung (4) in einem stabförmigen Modulgehäuse (10) angeordnet sind,
**dadurch gekennzeichnet,**
**daß** am Ende der Achse (11) des Rotationsantriebs (3) ein Stift (12) derart angeordnet ist, daß er bei der Rotation eine Kreisbewegung (13) beschreibt und in eine Wandlungswippe (14) eingreift, die mit einer senkrecht zur Rotationsachse (11) angeordneten Schwenkachse (15) am Modulgehäuse (10) gelagert ist, wobei der Stift (12) in die Wandlungswippe (14) beabstandet von der Schwenkachse (15) derart eingreift, daß er in der Richtung des Verlaufs der Schwenkachse (15) in der Wandlungswippe (14) gleitet, aber in Schwenkrichtung dieser eine Schwenkbewegung (16) vermittelt und daß die Pleuelstange (8) ebenfalls beabstandet von der Schwenkachse (15) an die Wandlungswippe (14) angelenkt ist, wobei eine weitere Anlenkung (9') an die Antriebsstange (9), die längs geführt ist, der Übertragung der Schwenkbewegung (16) und Umwandlung in die Hubbewegung (6) dient.

2. Antrieb (2) nach Anspruch 1,
**dadurch gekennzeichnet,**
**daß** die Schwenkachse (15) der Wandlungswippe (14), der Eingriff des Stifts (12) und die Anlenkung (17) der Pleuelstange (8) ein Dreieck bilden.

3. Antrieb (2) nach Anspruch 1 oder 2,
**dadurch gekennzeichnet,**
**daß** der Stift (12) drehbar gelagert ist.

4. Antrieb (2) nach Anspruch 1, 2 oder 3,
**dadurch gekennzeichnet,**
**daß** der Eingriff des Stifts (12) in die Wandlungswippe (14) ein in derselben Richtung wie die Schwenkachse (15) ausgerichtetes Langloch (18) ist.

5. Antrieb (2) nach Anspruch 1 oder 2,
**dadurch gekennzeichnet,**
**daß** der Stift (12) in eine Bohrung eines in der Wandlungswippe (14) gelagerten Schiebestifts (19) eingreift, wobei der Schiebestift (19), der in derselben Richtung wie die Schwenkachse (15) verläuft, verschiebbar in der Wandlungswippe (14) gelagert ist.

6. Mikronadelvorrichtung (1) mit einem Antrieb (2) nach einem der Ansprüche 1 bis 5,
**dadurch gekennzeichnet,**
**daß** das stabförmige Modulgehäuse (10) in einem stabförmigen Vorrichtungsgehäuse (20) angeordnet ist und daß eine Stichtiefeeinstellhülse (21) durch ein Gewinde (22) derart mit dem Vorrichtungsgehäuse (20) in Wirkverbindung steht, daß durch Drehung der Stichtiefeeinstellhülse (21) der Austrittshub (23) der Mikronadeln (7) aus der Nadelaustrittsöffnung (24) einstellbar ist.

7. Mikronadelvorrichtung (1) nach Anspruch 6,
**dadurch gekennzeichnet,**
**daß** der Antrieb (2) mit einem hinteren Teil des Vorrichtungsgehäuses (20") verbunden ist und daß die Stichtiefeeinstellhülse (21) auf einen vorderen Teil des Vorrichtungsgehäuses (20') wirkt, das mit dem hinteren Teil des Vorrichtungsgehäuses (20") verschiebbar aber drehfest verbunden ist.

8. Mikronadelvorrichtung (1) nach Anspruch 7,
**dadurch gekennzeichnet,**
**daß** das vordere Teil des Vorrichtungsgehäuses (20') ein Nadelführungsteil (26) und einen darin verschiebbar, aber drehfest gelagerten Nadelhalter (25) aufweist.

9. Mikronadelvorrichtung (1) nach Anspruch 8,
**dadurch gekennzeichnet,**
**daß** der Nadelhalter (25) mit der Nadelaustrittsöffnung (24) zur Aufnahme von in einer Reihe (29) angeordneten Mikronadeln (7) ausgebildet sind.

10. Mikronadelvorrichtung (1) nach einem der Ansprüche 6 bis 9,
**dadurch gekennzeichnet,**
**daß** das Nadelführungsteil (26) und der Nadelhalter (25) mit den Mikronadeln (7) in einem Auswechselmodul (27) angeordnet und an der übrigen Mikronadelvorrichtung (1) lösbar befestigt sind, wobei der Nadelhalter (25) mit der Antriebsstange (9) mittels einer Kupplung (28) verbindbar ist.
